# NEW EUROPEAN PATENT SPECIFICATION

(11) **EP 0 219 922 B2**
(45) Date of publication and mention of the opposition decision: **28.08.2002**
(45) Mention of the grant of the patent: 24.07.1991
(21) Application number: 86301513.7
(22) Date of filing: 04.03.1986
(51) Int. Cl.: A61K 9/50, A61K 31/70

(54) **Anthracycline antineoplastic agents encapsulated in phospholipid micellular particles**
In mizellulare phospholipidische Partikel eingekapselte antineoplastische Anthracyclinmittel
Agents antinéoplastiques à base d'anthracycline encapsulés dans des particules micellulaires phospholipidiques

(30) Priority: 15.10.1985 US 787535
(43) Date of publication of application: 29.04.1987
(73) Proprietor: VESTAR, INC., San Dimas California 91773 (US)
(72) Inventor: Forssen, Eric, Northridge, CA 91325 (US)
(74) Representative: Goldin, Douglas Michael

(56) References cited:
- WO-A-82/03769
- GB-A- 2 002 319
- US-A- 4 515 736
- CHEMICAL ABSTRACTS, vol. 93, no. 5, 4th August 1980, page 84, abstract no. 37237d, Columbus, Ohio, US; A. RAHMAN et al.: "Liposomal protection of adriamycin-induced cardiotoxicity in mice", & CANCER 1980, 40(5), 1532-7
- G. GREGORIADIS: "Liposome Technology", vol. 1: "Preparation of liposomes", 1984, pages 197-219, CRC Press Inc., Boca Raton, US; G. STRAUSS: "Freezing and thawing of liposome suspensions"
- "Preparation and characterization of doxorubicin containing liposomes I", D.J.A. Crommelin et al, Int. Journal of Pharmaceutics 16 (1983) 79-92
- "Preparation and characterization of doxorubicin containing liposomes II", D.J.A. Crommelin et al, Int. Journal of Pharmaceutics 17 (1983) 135-144
- "Preparation of Lipid Vesicles Containing High Level of Entrapped Radioactive Cations", Marcia R. Mauk et al, Analytical Biochemistry 94, 302-307 (1979) 302-307

## Description

### FIELD OF INVENTION

This invention relates to compositions consisting of phospholipid encapsulated anthracycline anti-neoplastic agents. In another aspect it relates to such compositions for use in delivering chemotherapeutic agents to tumors in a body.

### BACKGROUND

The anthracycline compounds including daunorubicin (also known as daunomycin), doxorubicin (also known as Adriamycin) AD-32 (N-Trifluoro-acetyl doxorubicin-14-valerate) and Aclacinomycin A are currently of great clinical interest for the treatment of tumors, including most leukemias and solid tumors. Structurally, these compounds consist of a hydrophobic tetracycline ring system coupled to an amino sugar through a glycoside linkage. These anthracycline agents associate with phosphate containing materials, and exhibit a high affinity for example, with cardiolipin. These compounds have been shown to exhibit marked activity against a wide variety of neoplasms. However, the clinical use of these drugs in humans has been limited by the chronic toxic effect of the drugs on heart tissue. Children, for example, are highly susceptible to doxorubicin-induced congestive heart failure. Mosijezuk, et al., Cancer, 44, p. 1582-1587 (1979). Long-term administration of such drugs leads to an increased risk of cardiomyopathy. Lefrak et al., Cancer, 32, p. 302-314 (1973).

Phospholipid micellular particles in the form of unilamellar vesicles known as liposomes, have received increasing attention as possible carriers for anthracycline drugs. Certain formulations have been shown to increase antitumor activity, alter in vivo tissue distribution and decrease toxicity. Difficulties have been encountered in producing encapsulated anthracyclines. In part this has been due to surfactant or to the detergent-like effect these compounds exert on phospholipid vesicle bilayer causing leakage and creating vesicle instability. Other problems have been the aggregation of such vesicles during storage. In addition, the efficiency of entrapment of previous formulations of encapsulated anthracyclines has been low, and has been reported to be between 5 and 65%. Forssen and Tokes, Cancer Res. 43, p.546-550 (1983). Gabizon et al., Cancer Res. 43, p. 4730-3745 (1983); and Gabizon et al., Br. J. Cancer 51, p. 681-689 (1985). Thus it has not been possible to achieve large scale production of stable, encapsulated anthracyclines for therapeutic purposes.

U.S.-A-4,515,736 describes a method in which liposome dispersions are dried in the presence of a material to be encapsulated. As drying occurs, the individual liposomes fuse to form multilamellar structures which capture the material between the lipid lamellae. Upon rehydration, lipid vesicles form which encapsulate the material.

Chemical Abstracts, Volume 93, No. 5, page 84, Abstract No. 37237d describes the formation of liposomes containing adriamycin which were used on the date of production after suspension in PBS.

GB-A-2,002,319 and Gregoriadis, Liposome Technology, Volume I, pages 197-219, CRC Press Inc., both describe the use of various materials including sugar as a cryoprotective agent when aqueous dispersions of liposomes are dehydrated.

The present invention provides improved formulations for encapsulating anthracycline, anti-neoplastic agents in phospholipid micellular particles and improved formulations of encapculated antineoplastic agents for use in a method of providing decreased cardiotoxicity and increased antitumour efficacy in humans.

### SUMMARY OF THE INVENTION

Compositions comprising Anthracycline neoplastic agents encapsulated in phospholipid micellular particles in the form of unilamellar vesicles about 45 to 55nm in diameter consisting of anionic and neutral phospholipids and cholesterol are described. The particles are suspended in a low ionic strength aqueous phase containing a sugar, such as a 5% dextrose solution, and exhibit a greater than 90% trapping efficiency of the anthracycline anti-neoplastic agent. Phospholipids which may be used include phosphatidyl glycerol, phosphatidyl choline, phosphatidyl serine, phosphatic acid and phosphatidyl inositol. A preferred anionic phospholipid is distearyl phosphatidyl glycerol. A preferred composition is daunorubicin, distearyl phosphatidyl glycerol, distearyl phosphatidyl choline and cholesterol. In one embodiment the ratio of these components is preferably from about 1:2:0:0 to about 1:4:20:20. Particularly preferred embodiments are ratios of 1:4:5:6 or 1:1.5:7:0 in an aqueous phase of a monosaccharide (e.g. dextrose) or a disaccharide (e.g. lactose, sucrose). The pH of the suspending solution is preferably between 4.0 to about 8.0. These compositions may be administered in multiple doses to a human subject to treat tumors.

### BRIEF DESCRIPTION OF THE DRAWINGS

Figure 1 illustrates in in vivo levels of C-14 labeled daunorubicin, free and vesicle entrapped, in the blood in mice at 1, 4, 24 and 48 hours.
Figure 2 illustrates the in vivo levels of C-14 labeled daunorubicin, free and vesicle entrapped, in tumors in mice at 1, 4, 24 and 48 hours.
Figure 3 illustrates the in vivo of C-14 labeled daunorubicin, free and vesicle entrapped, in heart tissue in mice at 1, 4, 24 and 48 hours.
Figure 4 illustrates the in vivo hepatic levels of C-14 labeled daunorubicin, free and vesicle entrapped, in mice at 1, 4, 24 and 48 hours.
Figure 5 depicts the rate of survival in mice bearing tumors treated on day 3 with a single dose of free or vesicle-entrapped daunorubicin.
Figure 6 shows the effect on tumor volume of single doses of daunorubicin, free or vesicle-entrapped, administered to mice bearing tumors.
Figure 7 illustrates the effect on tumor volume of multiple (20 mg/kg) doses of free or vesicle entrapped daunorubicin in mice bearing tumors.
Figure 8 depicts the survival rate for mice bearing tumors and receiving multiple doses (20 mg/kg) of free or vesicle entrapped daunorubicin.

### DETAILED DESCRIPTION OF THE INVENTION

As indicated above, according to this invention, encapsulation and improved delivery of anthracycline agents useful in treating tumors in humans is achieved using compositions containing micellular particles, in the form of small, unilamellar vesicles, consisting of phospholipids, cholesterol and an anthracycline agent and suspending such micellular particles in a low-ionic strength aqueous phase in which a monosaccharide, disaccharide, or other sugar is dissolved.

That anthracyclines exhibit a high affinity for the phospholipid cardiolipin appears to be of particular importance for mediating the biological activities of these drugs. Cardiolipin, however, is not a desirable constituent for phospholipid vesicles, in spite of its high affinity for anthracyclines, because when interacting with an anthracycline such as daunorubicin, it forms micelles which destabilize the bilayer structure of encapsulating micellular particles such as liposomes. Cardiolipin is also known to be highly antigenic in nature when incorporated in liposome membranes, such that it may cause an increased immunogenic response when injected into a body.

As noted above, one of the difficulties associated with the entrapment of anthracycline agents in phospholipid micellular particles is their amphiphilic nature causing the molecules to attempt to partition nearly equally between aqueous and lipid media. This partitioning causes these drugs to easily leak out from lipid membranes and to disrupt the membranes, destroying the bilayer of structures such as vesicles. An advantage of using a phospholipid such as DSPG is that it has an anionic charge which can be used to cancel the positive charge on the anthracycline molecule permitting production of neutral vesicles, which resist disruption and leakage. Furthermore, the use of a low-ionic strength aqueous phase to suspend the vesicles improves vesicle stability because it inhibits vesicle aggregation.

An additional advantage of using such negatively charged phospholipids is that the cancellation of the charge on the anthracycline molecule permits the formation of a water insoluble salt between the phospholipid and the anthracycline. This complex increases the affinity of the drug for the hydrophobic bilayer of the vesicle. While an anthracycline, such as daunorubicin, will bind fairly strongly to a negative phospholipid such as DSPG with a binding constant of approximately 10⁵M⁻¹, its affinity for binding to DNA in a cell is much greater, on the order of 2 x 10⁶M⁻¹ such that the drug will be able to be released from the DSPG and to complex with DNA present in the target tumor cells.

The micellular particles of this invention are in the form of small (45-55 nanometers in diameter) unilamellar phospholipid vesicles, prepared by sonication as described by M. R. Mauk and R. C. Gamble, Anal. Bioc., 94, p. 302-307 (1979), or by microemulsification using the procedures described in EP-A-0190050 These vesicles exhibit a high efficiency of entrapment (greater than 90%) of the anthracycline agent, a low tendency to aggregate, and a good storage life (about 90% particles intact after two weeks). One advantage of the higher entrapment efficiency is that the step of separating free drug from entrapped drug after loading procedures may be eliminated, thus simplifying manufacture.

Adjustment of pH is an additional factor for maximum drug entrapment with an optimal range of from about pH 4.0 to 8.0. A suitable buffer for maintaining pH is TRIS (Tromethamine or 2-amino-2-hydroxymethyl-1, 3-propanediol) since it can readily be buffered over a pH range of 7 to 9. Other buffering agents may include sodium acetate and sodium benzoate.

It has been found in the present invention that by using anionic phospholipids such as distearyl phosphatidyl glycerol (DSPG) with neutral phospholipids such as distearyl phosphatidyl choline (hereafter DSPC), the partitioning of an anthracycline agent such as daunorubicin (hereafter DAU) into the lipid phase may be increased leading to a much increased percent of entrapment of the agent in the micellular particle and more stable particles. It has also been found that the incorporation of cholesterol (CHOL) leads to improved stability of the particles encapsulating the anthracycline. The stability of these compositions is further enhanced by suspending the particles in a low-ionic strength solution such as a 5% dextrose solution.

A preferred formulation is DAU:OSPG:DSPC:CHOL in a molar ratio of 1:4:5:6. The range of these components may be from about 1:2:0:0 to about 1:4:20:20. Other embodiments include ratios of 1:1.5:7:0, 1:4:5:4, 1:2:6:0, 1:2:20:0 and 1:2:6:1. Preferably, the DSPG is present in at least a fifty percent (molar) excess relative to DAU. It appears however, that there is no upper limit to the amount of DSPG (or other anionic phospholipid) which may be incorporated. The preferred amount of cholesterol which may be incorporated is approximately equal to the amount of DSPG present in from 0 to 20 times the amount of DAU present. Other anionic phospholipids, for example phosphatidyl serine and phosphatidic acid, may be used. Because neutral vesicles appear to be more effective as deliver vehicles to tumors (see, Mauk and Gamble) it may be desirable to select a ratio of phospholipid components which minimise the net negative charge of the vesicles while maintaining the physical integrity of the vesicles' structure by preserving the stability of the anthracycline in the bilayer. Thus, for certain applications a ratio of DAU:DSPG of 1:1.5 may be preferred.

To prepare vesicles, the lipids and anthracycline agent, for example, daunorubicin, to be used for vesicle preparation are weighed out in the desired ratios and are either dissolved in an organic solvent such as methanol or chloroform or kept until use as dry powders. If a solvent is used, it must be removed prior to the addition of the aqueous phase by evaporation, for example under argon, nitrogen or by application of a vacuum.

The aqueous phases preferred for formulation of anthracycline vesicles with high entrapment and maximum stability are low-ionic media, such as sugar solutions or de-ionized distilled water. A 5% dextrose in water solution at pH 7.4 is preferred. Other solutions such as a 9% lactose or 9% sucrose solution in water may also be used. Such solutions minimize drug leakage from vesicles and decrease vesicle aggregation, and are well suited for parenteral use, for example human intravenous injection.

The example which follows describes the preparation, characterization and in vivo chemotherapeutic application in an animal model for a vesicle formulation of this invention.

### EXAMPLE

### DAUNORUBICIN VESICLES

### Preparation of Vesicles Encapsulating Daunorubicin

Phospholipid vesicles were prepared using distearyl phosphatidyl glycerol (DSPG), distearyl phosphatidyl choline (DSPC), cholesterol (CHOL), and daunorubicin (DAU) in a molar ratio of DAU:DSPG:DSPC:CHOL of 1:4:5:6.

The lipids were obtained from Avanti Polar Lipids, (Birmingham, Alabama) and the daunorubicin was obtained from Sigma Chemical Co., (St. Louis, Missouri). These compounds were weighed out in the desired ratios and were dissolved in the organic solvent chloroform. The solvent was removed prior to addition of the aqueous phase by evaporation under nitrogen gas followed by vacuum. The non-ionic aqueous phase consisting of 5% dextrose solution in water, pH adjusted. to 7.4 with NaOH was added to the lipid mixture and the solution was heated in a water bath at 60° to 70° C for 1 to 3 minutes then vigorously agitated to form a suspension of the drug-lipid mix. This step was repeated until all the material had been suspended in the aqueous phase. This mixture was then sonicated using a needle probe sonicator (Sonics and Materials, Danbury, Conn.), at an output control setting of 1-2 (on a scale of 10). The same was sonicated until clear, about 2-5 minutes for a 5 ml sample. During sonication the mixture was heated at 10° to 80° C in a water bath. Following sonication, the sample was centrifuged to remove all particulate matter.

### Characterization of Vesicles Encapsulating Daunorubicin

The vesicles containing daunorubicin, prepared as described above, were characterized for size (diameter) and entrapment efficiency following preparations. Vesicle sizing was performed using a Laser Particle Sizer Model 200 (Nicomp Instruments, Santa Barbara, California) and was determined to be in the range of 45 to 55 nanometers in diameter.

The efficiency of association of daunorubicin within the vesicles was estimated using Sephadex G 50 gel-filtration to separate free from entrapped daunorubicin. Using the above formulations, 95-100% of the daunorubicin was found to be associated with the vesicles. Due to this high association, additional separation steps were unnecessary to remove free drug.

The daunorubicin vesicles prepared as described above were examined using HPLC and were found to be stable as indicated by the lack of chemical decomposition comparing freshly sonicated vesicles with those left at room temperature for two weeks. In addition, when a 2 ml sample of these vesicles were frozen in dry ice and later thawed at 65° C, the vesicles maintained their original size as determined by light scattering using the Laser Particle Sizer, and also retained all of the previously entrapped daunorubicin as determined by Sephadex gel filtration. Finally, incubation of Indium-III loaded daunorubicin vesicles in serum at 37° C for 24 hours following the procedures described by Mauk and Gamble, Anal. Bioc., 94, p. 302-307 (1979), for loading In-III in phospholipid vesicles, demonstrated no loss in entrapped In-III had occurred as determined by x-ray perturbed angular correlation ("PAC") (no decline in G₂₂).

### Biodistribution of C-14 Labeled Daunorubicin Vesicles

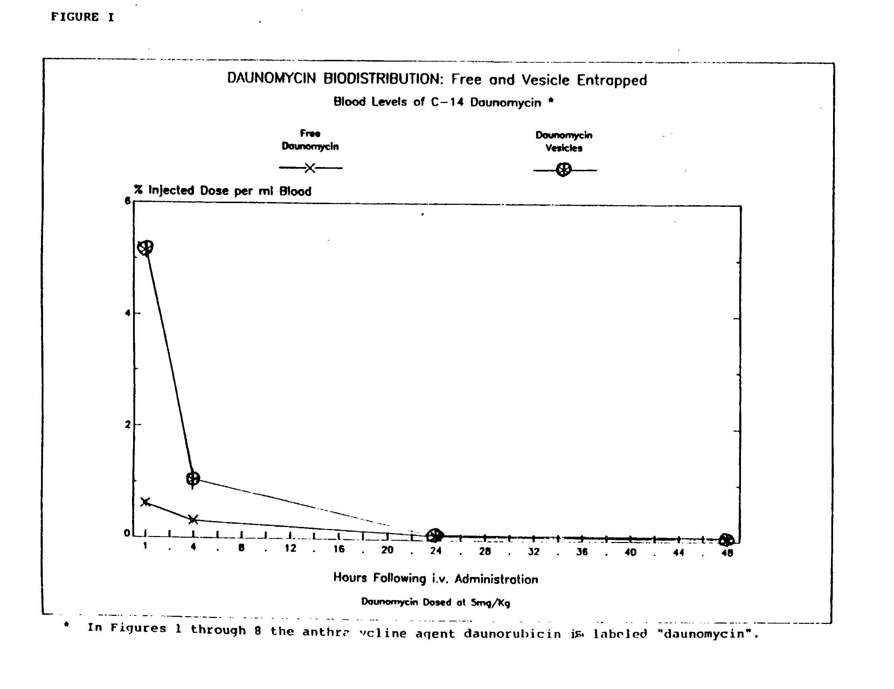

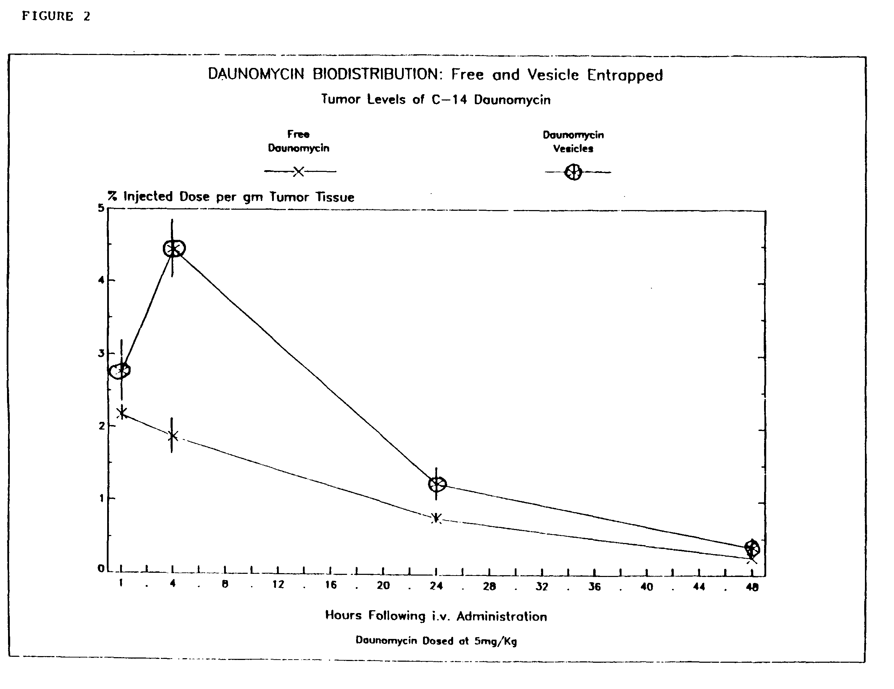

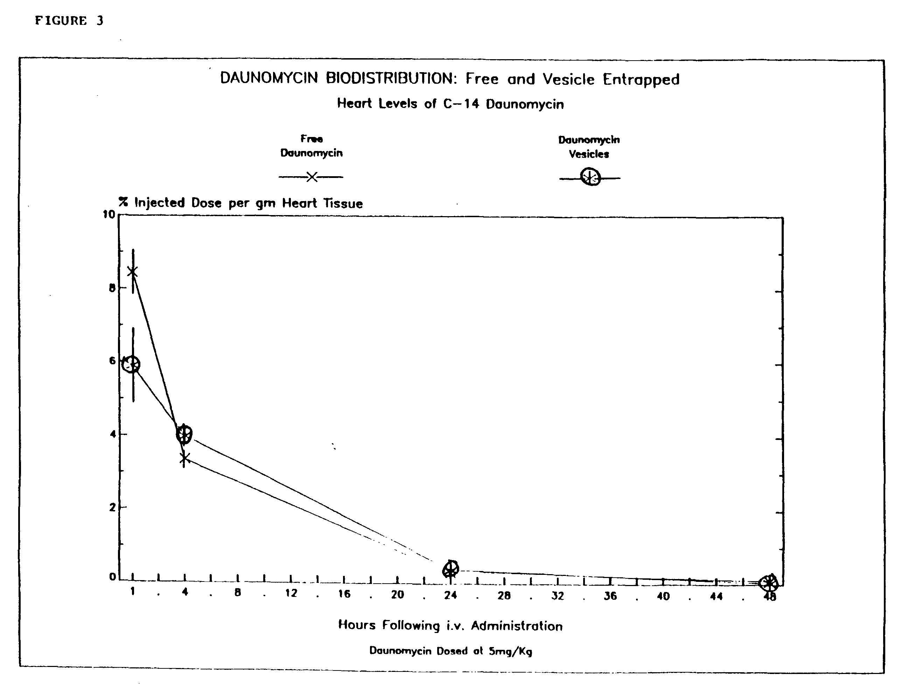

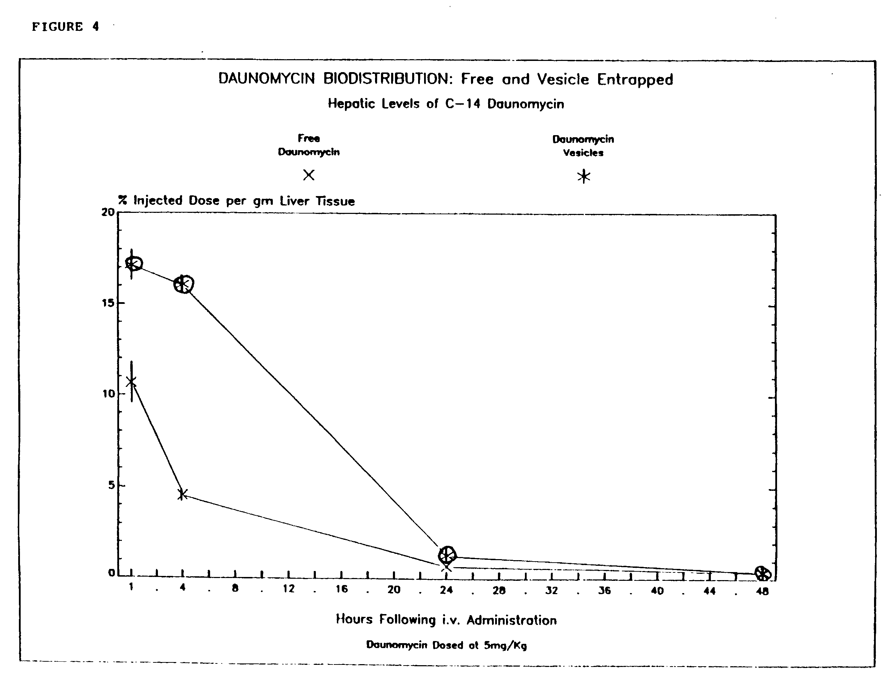

Biodistribution studies of C-14 labeled daunorubicin, both free and vesicle entrapped, were conducted using a daunorubicin dose of 5 mg/kg in CD₂F₁ mice bearing intradermal P-1798 lymphosarcoma solid tumor. Time points were taken at 1, 4, 24, and 48 hours. The results are presented in FIG. 1 through 4 showing that daunorubicin vesicles remain in the blood for longer periods of time than free drug and that in tumor tissue the level of vesicle encapsulated daunorubicin was significantly higher than free daunorubicin.

### Toxicity

It appears that daunorubicin vesicles are not more toxic and are most likely less toxic than unencapsulated drug in animals bearing tumors as determined by survival in a small sample of mice using doses of 10, 20 and 30 mg/kg. In this limited study, toxicity induced deaths occurred only for the high dose unencapsulated daunorubicin (30 mg/kg), at a 100% rate. In contrast, no deaths occurred in mice receiving an equal dose of vesicle encapsulated daunorubicin.

### Chemotherapeutic Efficacy of Daunorubicin Vesicles

CD₂F₁ mice implanted with intradermal p-1798 solid lymphosarcoma received free and vesicle-encapsulated daunorubicin in a single dose injection of 20 mg/kg, and in multiple dosages of 5, 10 and 20 mg/kg.

In the first investigation, groups of 10 mice received free daunorubicin or daunorubicin-vesicles in 20 mg/kg single doses at three or four days following tumor implantation. Tumors were measured using calipers and the survival over time of treated and control mice was recorded. Controls consisted of injections of a 5% dextrose in water solution at doses of 0,0021/0.02kg mouse [2 ml/20 g mouse].

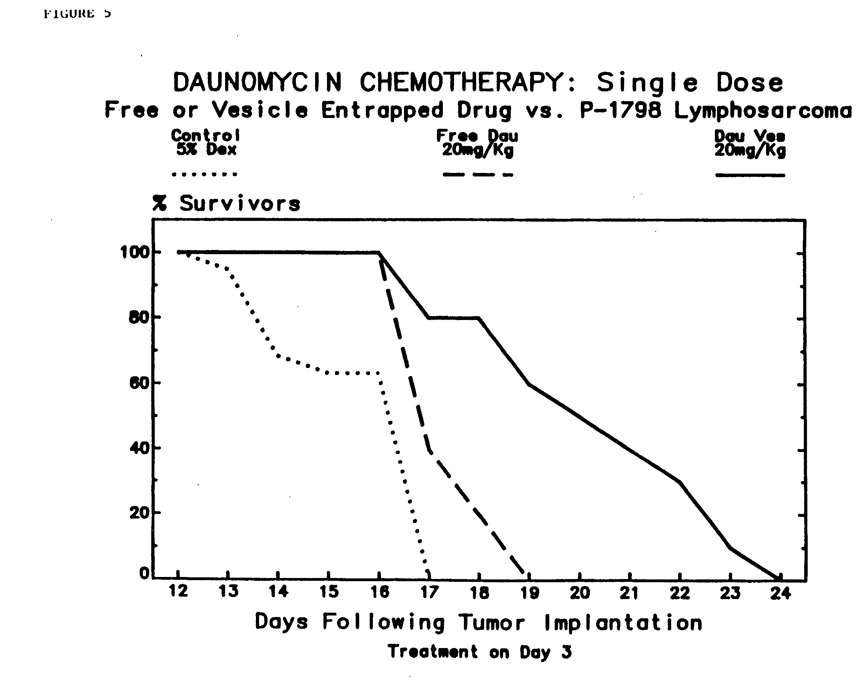

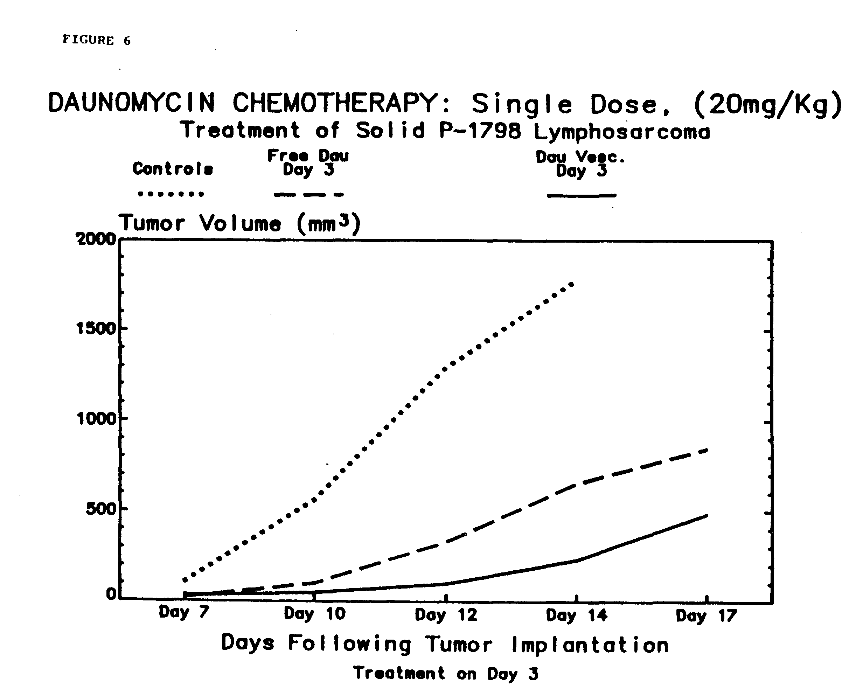

Representative results of these investigations are shown in FIG. 5 for treatment commencing on day 3 after tumor implantation. Typically, with tumor metatastasis, mice with tumors die within 14-17 days. Survival times for daunorubicin-vesicle treated mice increased in comparison to free drug. The median life span of mice injected with daunorubicin-vesicles was 21 days. All mice receiving free or vesicle entrapped daunorubicin demonstrated significant inhibition of tumor growth compared with untreated controls. Mice receiving vesicle entrapped daunorubicin had less tumor growth than those receiving free doses, as depicted in FIG. 6.

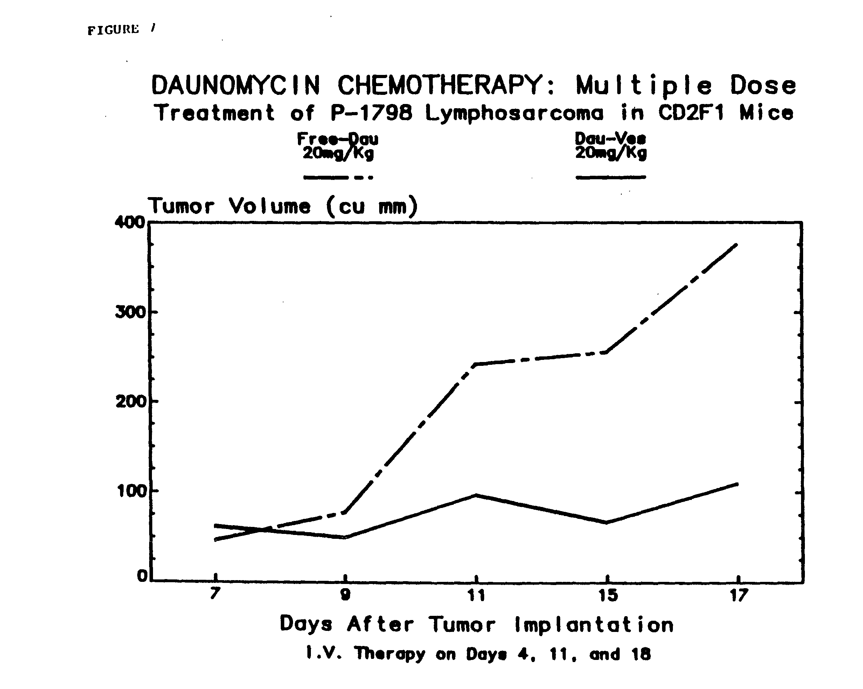

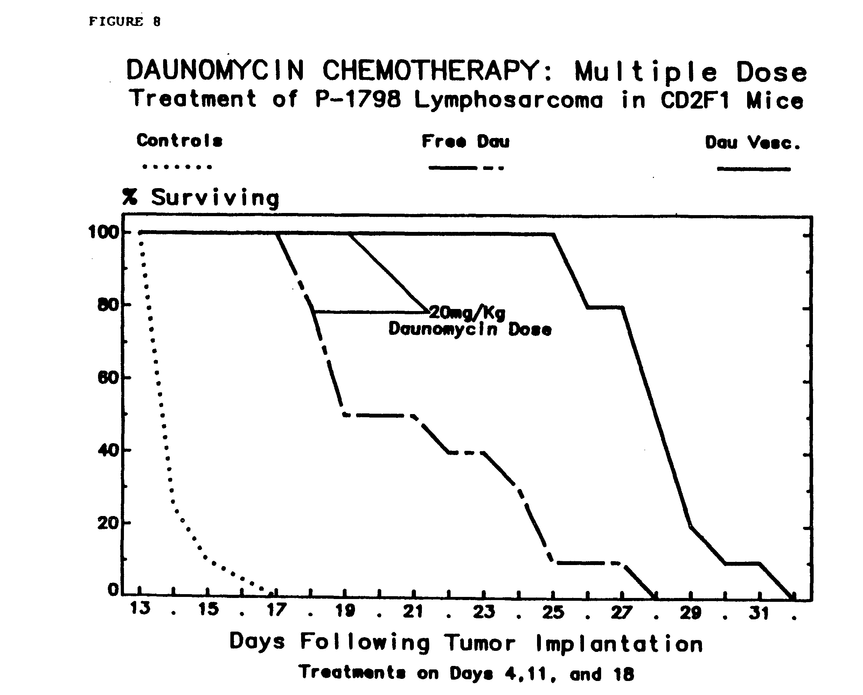

In a second study of chemotherapeutic effects of injections of daunorubicin-vesicles, groups of ten mice received multiple doses of 5, 10 and mg/kg. Treatments were initiated on day 4 and followed at weekly intervals (day 11, day 18) for a total of three doses. Body weight and tumor size were monitored during the study. As shown in FIG. 7, at 20 mg/kg significant inhibition of tumor growth occurred in daunorubicin-vesicle treated mice compared with those treated with free drug. Survival times were investigated in a group of 19 mice and as indicated in FIG. 8 following tumor implantation were significantly increased for daunorubicin-vesicle treated mice relative to free drug at doses of 20 mg/kg.

These results clearly demonstrate the usefulness and efficacy of the vesicle formulations of the present invention as improved vehicles for delivering anthracyclines to tumors in a body.

## Claims

1. A composition comprising an anthracycline anti-neoplastic agent encapsulated in phospholipid micellular particles in the form of unilamellar vesicles about 45 to 55 nanometers in diameter consisting of anionic and neutral phospholipids and cholesterol, said particles suspended in a low ionic strength aqueous phase containing sugar and exhibiting a greater than 90% trapping efficiency of the anthracycline anti-neoplastic agent.

2. A composition according to claim 1 wherein the sugar is dextrose.

3. A composition according to Claim 1 wherein the aqueous phase is a sugar solution containing 5% dextrose.

4. A composition according to any one of the preceding claims wherein the phospholipid is at least one selected from phosphatidyl glycerol, phosphatidyl choline, phosphatidyl serine, phosphatidic acid and phosphatidyl inositol.

5. A composition according to claim 4 wherein the phospholipids are distearyl phosphatidyl glycerol and distearyl phosphatidyl choline.

6. The composition according to claim 5 comprising the anthracycline agent, distearyl phosphatidyl glycerol, distearyl phosphatidyl choline and cholesterol in a molar ratio ranging from 1:2:0:0 to 1:4:20:20.

7. A composition according to claim 6 wherein the anthracycline agent, distearyl phosphatidyl glycerol, distearyl phosphatidyl choline and cholesterol are in the molar ratio of 1:4:5:6.

8. A compound according to claim 5 wherein the anthracycline agent, distearyl phosphatidyl glycerol, distearyl phosphatidyl choline and cholesterol are in the molar ratio of 1:1,5:4:0.

9. A composition according to claim 7 wherein the pH of the suspending solution is in the range of from pH 4.0 to 8.0.

10. A composition according to claim 9, wherein the pH is 7.4.

11. A composition according to any one of the preceding claims wherein the anthracycline agent is daunorubicin, doxorubicin, N-Trifluoroacetyl doxorubicin-14-valerate and Aclacinomycin A.

12. A composition according to any one of the preceding claims wherein the anthracycline agent is daunorubicin.

13. A composition according to any one of the preceding claims for use in a method of therapy practised on the human body.

14. A composition according to claim 13 for use in a method for treating neoplastic tumours in a human body by parenterally administering multiple doses of the composition into a human subject.

15. A composition according to claim 14 for use in a method wherein the administration is by intravenous injection.

16. A method of preparing a composition as claimed in any one of claims 1 to 15 comprising an anthracycline anti-neoplastic agent by encapsulating the anti-neoplastic agent in phospholipid micellular particles consisting of anionic and neutral phospholipids and cholesterol, said particles suspended in a low ionic strength aqueous phase containing sugar.

17. A method according to claim 16 wherein the agent in the micellular particles is as defined in any one of claims 2 to 12.

## Patentansprüche

1. Zusammensetzung umfassend ein antineoplastisches Anthracyclin-Agens, das in Phospholipid-Mizellteilchen in Form unilamellarer Vesikel von etwa 45 bis 55 nm Durchmesser eingekapselt ist, die aus anionischen und neutralen Phospholipiden und Cholesterin bestehen, wobei diese Teilchen in einer Zucker enthaltenden wäßrigen Phase geringer lonenstärke suspendiert sind und eine Einschlußeffizienz des antineoplastischen Anthracyclin-Agens von über 90 % aufweisen.

2. Zusammensetzung nach Anspruch 1,
**dadurch gekennzeichnet,**
**daß** der Zucker Dextrose ist.

3. Zusammensetzung nach Anspruch 1,
**dadurch gekennzeichnet,**
**daß** die wäßrige Phase eine Zuckerlösung ist, die 5 % Dextrose enthält.

4. Zusammensetzung nach einem der vorhergehenden Ansprüche,
**dadurch gekennzeichnet,**
**daß** das Phospholipid mindestens eines ist, ausgewählt aus Phosphatidylglycerin, Phosphatidylcholin, Phosphatidylserin, Phosphatidsäure und Phosphatidylinosit.

5. Zusammensetzung nach Anspruch 4,
**dadurch gekennzeichnet,**
**daß** die Phospholipide Distearylphosphatidylglycerin und Distearylphosphatidylcholin sind.

6. Zusammensetzung nach Anspruch 5, umfassend das Anthracyclin-Agens, Distearylphosphatidylglycerin, Distearylphosphatidylcholin und Cholesterin in einem Molverhältnis von 1:2:0:0 bis 1:4:20:20.

7. Zusammensetzung nach Anspruch 6,
**dadurch gekennzeichnet,**
**daß** das Anthracyclin-Agens, Distearylphosphatidylglycerin, Distearylphosphatidylcholin und Cholesterin im Molverhältnis 1:4:5:6 vorliegen.

8. Zusammensetzung nach Anspruch 5,
**dadurch gekennzeichnet,**
**daß** das Anthracyclin-Agens, Distearylphosphatidylglycerin, Distearylphosphatidylcholin und Cholesterin im Molverhältnis 1:1:5:4:0 vorliegen.

9. Zusammensetzung nach Anspruch 7,
**dadurch gekennzeichnet,**
**daß** der pH-Wert der Suspensionslösung im Bereich von pH = 4,0 bis 8,0 liegt.

10. Zusammensetzung nach Anspruch 9,
**dadurch gekennzeichnet,**
**daß** der pH=Wert 7,4 ist.

11. Zusammensetzung nach einem der vorhergehenden Ansprüche,
**dadurch gekennzeichnet,**
**daß** das Anthracyclin-Agens Daunorubicin, Doxorubicin, N-Trifluoracetyldoxorubicin-14-valeriat und Aclacinomycin A ist.

12. Zusammensetzung nach einem der vorhergehenden Ansprüche,
**dadurch gekennzeichnet,**
**daß** das Anthracyclin-Agens Daunorubicin ist.

13. Zusammensetzung nach einem der vorhergehenden Ansprüche zur Verwendung in einen am menschlichen Körper durchgeführten Therapieverfahren.

14. Zusammensetzung nach Anspruch 13 zur Verwendung in einem Verfahren zur Behandlung neoplastischer Tumore in einem menschlichen Körper durch parenterale Verabreichung mehrfacher Dosen der Zusammensetzung an einen Menschen.

15. Zusammensetzung nach Anspruch 14 zur Verwendung in einem Verfahren, bei dem die Verabreichung durch intravenöse Injektion erfolgt.

16. Verfahren zur Herstellung einer Zusammensetzung nach einem der Ansprüche 1 bis 15, die ein antineoplastisches Anthracyclin-Agens umfaßt, durch Einkapselung des neoplastischen Agens in Phospholipid-Mizellteilchen, die aus anionischen und neutralen Phospholipiden und Cholesterin bestehen, wobei die Teilchen in einer Zucker enthaltenden wäßrigen Phase geringer Ionenstärke suspendiert sind.

17. Verfahren nach Anspruch 16,
**dadurch gekennzeichnet,**
**daß** das Agens in den Mizellteilchen ein nach einem der Ansprüche 2 bis 12 definiertes ist.

## Revendications

1. Composition comprenant un agent anti-néoplastique à base d'anthracycline, encapsulé dans des particules micellaires phospholipidiques sous la forme de vésicules unilamellaires ayant environ 45 à 55 nanomètres de diamètre constituées de phospholipides anioniques et neutres et de cholestérol, lesdites particules étant en suspension dans un phase aqueuse de faible force ionique contenant un sucre et ayant un facteur de capture de l'agent antinéoplasique à base d'anthracycline supérieur à 90%.

2. Composition selon la revendication 1, dans laquelle le sucre est le dextrose.

3. Composition selon la revendication 1, dans laquelle la phase aqueuse est une solution de sucre contenant 5% de dextrose.

4. Composition selon l'une quelconque des revendications précédentes, dans laquelle le phospholipide est au moins l'un des composés choisi parmi le phosphatidyl-glycérol, la phosphatidylcholine, la phosphatidyl-sérine, l'acide phosphatidique et le phosphatidyl-inositol.

5. Composition selon la revendication 4, dans laquelle les phospholipides sont le distéaryl-phosphatidyl-glycérol et la distéaryl-phosphatidyl-choline.

6. Composition selon la revendication 5, comprenant l'agent à base d'anthracycline, le distéaryl-phosphatidyl-glycérol, la distéaryl-phosphatidyl-choline et le cholestérol avec un rapport molaire dans la gamme allant de 1:2:0:0 à 1:4:20:20.

7. Composition selon la revendication 6, dans laquelle l'agent à base d'anthracycline, le distéaryl-phosphatidyl-glycérol, la distéaryl-phosphatidyl-choline et le cholestérol sont dans le rapport molaire de 1:4:5:6.

8. Composition selon la revendication 5, dans laquelle l'agent à base d'anthracycline, le distéaryl-phosphatidyl-glycérol, la distéaryl-phosphatidyl-choline et le cholestérol sont dans le rapport molaire de 1:1,5:4:0.

9. Composition selon la revendication 7, dans laquelle le pH de la solution de suspension est dans la gamme de pH 4,0 à 8,0.

10. Composition selon la revendication 9, dans laquelle le pH est 7,4.

11. Composition selon l'une quelconque des revendications précédentes, dans laquelle l'agent à base d'anthracycline est la daunorubicine, la doxorubicine, le N-trifluoroacétyl-doxorubicin- 14-valérate et l'aclacinomycine A.

12. Composition selon l'une quelconque des revendications précédentes, dans laquelle l'agent à base d'anthracycline est la daunorubicine.

13. Composition selon l'une quelconque des revendications précédentes, destinée à l'utilisation dans une méthode thérapeutique appliquée au corps humain.

14. Composition selon la revendication 13, destinée à l'utilisation dans une méthode de traitement des tumeurs néoplasiques dans le corps humain, par administration par voie parentérale de doses multiplies de la composition à un sujet humain.

15. Composition selon la revendication 14, destinée à l'utilisation dans une méthode dans laquelle l'administration est effectuée par injection intraveineuse.

16. Procédé de préparation d'une composition telle que revendiquée dans l'une quelconque des revendication 1 à 15, comprenant un agent antinéoplasique à base d'anthracycline, par encapsulation de l'agent antinéoplasique dans des particules micellaires phospholipidiques constituées de phospholipides anioniques et neutres et de cholestérol, lesdites particules étant en suspension dans une phase aqueuse de force ionique faible, contenant un sucre.

17. Procédé selon la revendication 16, dans lequel l'agent contenu dans les particules micellaires est tel que défini dans l'une quelconque des revendications 2 à 12.
